(19) Europäisches Patentamt
European Patent Office
Office européen des brevets

(11) Veröffentlichungsnummer : **0 392 299 B1**

# (12) EUROPÄISCHE PATENTSCHRIFT

(45) Veröffentlichungstag der Patentschrift : **19.05.93 Patentblatt 93/20**

(51) Int. Cl.$^5$ : **C07C 209/62,** C07D 251/34, C08G 18/32

(21) Anmeldenummer : **90106216.6**

(22) Anmeldetag : **31.03.90**

## (54) Verfahren zur Herstellung von organischen Aminen, Polyamingemische und ihre Verwendung als Härter für Kunststoffvorläufer.

(30) Priorität : **14.04.89 DE 3912266**
**01.12.89 DE 3939699**

(43) Veröffentlichungstag der Anmeldung : **17.10.90 Patentblatt 90/42**

(45) Bekanntmachung des Hinweises auf die Patenterteilung : **19.05.93 Patentblatt 93/20**

(84) Benannte Vertragsstaaten : **AT BE CH DE DK ES FR GB IT LI NL SE**

(56) Entgegenhaltungen :
**DD-A- 128 980**
**DE-A- 3 039 600**
**FR-A- 1 415 317**
**FR-A- 2 378 748**

(56) Entgegenhaltungen :
**CHEMICAL ABSTRACTS, vol. 78, no. 23, June 11, 1973, Columbus, Ohio, US; NUKINA, SHOKO et al.: "Tris(3-aminopropyl)isocyanuric acid", p. 388, col. 2, abstr. no. 148 005m**
**CHEMICAL ABSTRACTS, vol. 80, no. 4, January 28, 1974,Columbus, Ohio, US; NUKINA, SHOKO et al.: "Thermosetting resin", p. 15 810, col. 2, abstr. no. 15808v**
**SYNTHESIS, November 1988, Stuttgart; F. ZARAGOZA-DOERWALD: "A simple and economic synthesis of monoacylated alkanediamines by thermal transamidation", p. 917, 918**

(73) Patentinhaber : **BAYER AG**
**W-5090 Leverkusen 1 Bayerwerk (DE)**

(72) Erfinder : **Scholl, Hans Joachim, Dr.**
**Am Feldrain 5**
**W-5000 Köln 80 (DE)**

EP 0 392 299 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein neues Verfahren zur Herstellung von organischen Aminen mit aliphatisch und/oder cycloaliphatisch gebundenen primären Aminogruppen aus den entsprechenden organischen Isocyanaten mit aliphatisch und/oder cycloaliphatisch gebundenen Isocyanatgruppen durch Formylierung der Isocyanatgruppen dieser Isocyanate und anschließende Überführung der resultierenden N-Formylgruppen in Aminogruppen, die besonders bevorzugten, hierdurch erstmals erhältlichen Polyamingemische bestehend aus N,N',N"-Tris-(6-aminohexyl)isocyanurat und seinen höheren Homologen, sowie die Verwendung der letztgenannten Gemische als Härter für Epoxid- oder Isocyanatgruppen aufweisende Kunststoffvorläufer.

Die Herstellung von primären Aminen durch hydrolytische Zersetzung der Isocyanatgruppen der entsprechenden Isocyanate ist bereits bekannt. So wird beispielsweise in EP-A-0 284 887 oder EP-A-0 285 948 ein entsprechendes Verfahren zur Herstellung von endständige Aminogruppen aufweisenden Prepolymeren aus den entsprechenden NCO-Prepolymeren beschrieben. Als Ausgangsmaterialien werden im Text dieser Veröffentlichungen sowohl NCO-Prepolymere mit aliphatisch als auch mit aromatisch gebundenen Isocyanatgruppen genannt, wobei allerdings jene mit aromatisch gebundenen Isocyanatgruppen bevorzugt sind. Tatsächlich ist das Verfahren dieser Vorveröffentlichungen auf NCO-Prepolymere mit aliphatisch gebundenen Isocyanatgruppen in der Praxis nicht anwendbar, weil, bedingt durch eine Vielzahl an Nebenreaktionen, unbrauchbare Produktgemische mit geringer Aminausbeute entstehen.

Die FR-A-1 415 317 (Beispiel 2) beschreibt ein anderes Verfahren zur Überführung von aromatisch gebundenen Isocyanatgruppen in die entsprechenden Aminogruppen, bei welchem die Isocyanatgruppen des Ausgangsmaterials mit Ameisensäure in formylierte Aminogruppen überführt werden, die dann durch alkalische oder saure Verseifung in die entsprechenden Aminogruppen überführt werden.

Wie jetzt gefunden wurde gelingt insbesondere die Herstellung von Aminen mit aliphatich und/oder cycloaliphatisch gebundenen Aminogruppen auf besonders einfache Weise durch Formylierung der Isocyanatgruppen von organischen Isocyanaten mit aliphatisch und/oder cycloaliphatisch gebundenen Isocyanatgruppen und Überführung der hierbei gebildeten formylierten Aminogruppen in freie Aminogruppen durch eine Transformylierungsreaktion, bei welcher die Formylgruppen unter Freisetzung der Aminogruppen auf die Aminogruppen eines anderen, im allgemeinen leichtflüchtigen primären Amins übertragen werden.

Wie auch nachstehend nochmals hervorgehoben wird, unterscheidet sich diese erfindungswesentliche Transformylierungsreaktion in einigen wesentlichen Punkten von der in Synthesis (1988), Seiten 917 - 918 beschriebenen Transamidierung.

Das nachstehend näher beschriebene erfindungsgemäße Verfahren erlaubt insbesondere erstmals die Herstellung von Polyamingemischen, die im wesentlichen aus N,N',N"-Tris-(6-aminohexyl)-isocyanurat und seinen höheren Homologen bestehen, und die besonders wertvolle Härter für Epoxid- oder Isocyanatgruppen aufweisende Kunststoffvorläufer darstellen.

Die JP-A-47 043 320 (vgl. Chem. Abstr. 80:15808 v (1974) erwähnt zwar "Tris(3-aminopropyl)-isocyanurat" als Härter für Epoxidharze, d.h. eine Verbindung, die strukturell einer Komponente ähnlich ist, die in den erfindungsgemäßen Polyamingemischen als Komponente a) vorliegt, jedoch wird weder auf die Herstellung der genannten Verbindung eingegangen noch sind Polyamingemische der nachstehend näher beschriebenen erfindungsgemäßen Art in der Vorveröffentlichung genannt.

Gegenstand der Erfindung ist ein Verfahren zur Herstellung von primären Aminen mit aliphatisch oder cycloaliphatisch gebundenen Aminogruppen durch Formylierung der Isocyanatgruppen von organischen Isocyanaten mit aliphatisch oder cycloaliphatisch gebundenen Isocyanatgruppen mit Ameisensäure und anschließende Überführung der N-Formylgruppen in Aminogruppen, dadurch gekennzeichnet, daß man die N-Formylgruppen durch eine Transformylierungsreaktion in Aminogruppen überführt. Gegenstand der Erfindung sind auch die besonders bevorzugten, nach diesen Verfahren erhältlichen Polyamingemische, die dadurch gekennzeichnet sind, daß sie im wesentlichen aus

a) 40 bis 90 Gew.-%, bezogen auf das Gesamtgewicht der Komponenten a) und b), N,N',N"-Tris-(6-aminohexyl)-isocyanurat und

b) 10 bis 60 Gew.-%, bezogen auf das Gesamtgewicht der Komponenten a) und b), an höheren, mehr als einen Isocyanuratring aufweisenden Homologen dieses Tri-amins bestehen.

Für das erfindungsgemäße Verfahren können beliebige organische Isocyanate mit aliphatisch und/oder cycloaliphatisch gebundenen primären Isocyanatgruppen eingesetzt werden. Geeignet sind beispielsweise

1. aliphatische oder cycloaliphatische Monoisocyanate mit insgesamt bis zu 19 Kohlenstoffatomen wie n-Hexylisocyanat, n-Dodecylisocyanat, n-Stearylisocyanat oder Cyclohexylisocyanat;

2. niedermolekulare organische Polyisocyanate eines maximalen Molekulargewichts von 399 wie beispielsweise Hexamethylendiisocyanat, 1-Isocyanato-3,3,5-trimethyl-5-isocyanatomethylcyclohexan oder 4,4'-Diisocyanato-dicyclohexylmethan;

3. höhermolekulare organische Polyisocyanate mit aliphatisch und/oder cycloaliphatisch gebundenen Isocyanatgruppen und einem Molekulargewicht von mindestens 400, vorzugsweise 400 bis 5000 wie z.B. NCO-Prepolymere aus überschüssigen Mengen der unter 2, genannten einfachen Diisocyanate und nieder- oder höhermolekularen organischen Polyhydroxylverbindungen der aus der Polyurethanchemie an sich bekannten Art, insbesondere Prepolymere aus den beispielhaft genannten Diisocyanaten und Polyether- oder Polyester-Polyolen eines über 400 liegenden Molekulargewichts, Biuretgruppen aufweisende Polyisocyanate wie beispielsweise N,N',N"-Tris-(6-isocyanatohexyl)-biuret bzw. seine Gemische mit seinen höheren Homologen oder Isocyanuratgruppen aufweisende Polyisocyanate auf Basis der unter 2. beispielhaft genannten einfachen Diisocyanate.

Die unter 3. genannten höhermolekularen Polyisocyanate sind gegenüber den unter 1. und 2. genannten niedermolekularen Ausgangsverbindungen bevorzugt. Besonders bevorzugt werden beim erfindungsgemäßen Verfahren Isocyanuratgruppen aufweisende Polyisocyanatgemische eingesetzt, die im wesentlichen aus

a) 40 bis 90 Gew.-%, bezogen auf das Gesamtgewicht der Komponenten a) und b), N,N',N"-Tris-(6-isocyanatohexyl)-isocyanurat und

b) 10 bis 60 Gew.-%, bezogen auf das Gesamtgewicht der Komponenten a) und b), an höheren, mehr als einen Isocyanuratring aufweisenden Homologen dieses Triisocyanats bestehend.

Die zuletztgenannten als Ausgangsmaterialien für das erfindungsgemäße Verfahren besonders bevorzugten Polyisocyanatgemische entstehen durch an sich bekannte Trimerisierung eines Teils der Isocyanatgruppen von Hexamethylendiisocyanat unter anschließender destillativer Entfernung von nicht umgesetztem Ausgangsdiisocyanat. Die Homologenverteilung in den resultierenden Polyisocyanatgemischen hängt insbesondere vom Trimerisierungsgrad während der Durchführung der Trimerisierungsreaktion ab. Unter "Trimerisierungsgrad" ist hierbei der Prozentsatz der im Ausgangsdiisocyant vorliegenden Isocyanatgruppen zu verstehen, die während der Trimerisierung abreagieren. Im allgemeinen liegt der Trimerisierungsgrad bei der Herstellung der Isocyanuratgruppen aufweisenden Polyisocyanatgemische bei 10 bis 40 %. Ein niederer Trimerisierungsgrad führt zu einem Polyisocyanatgemisch mit einem hohen Gehalt an Triisocyanat, während ein hoher Trimerisierungsgrad zu einem Polyisocyanatgemisch mit einem vergleichsweise hohen Gehalt an höheren Homologen führt.

Verfahren zur Herstellung derartiger Polyisocyanatgemische durch Teiltrimerisierung von Hexamethylendiisocyanat sind beispielsweise in US-PS 4 324 879, DE-OS 3 100 262 oder in Chem. Rev. 72, 477 (1972) beschrieben.

Bei der Durchführung des erfindungsgemäßen Verfahrens wird mindestens ein Äquivalent Ameisensäure pro NCO-Äquivalent eingesetzt. Vorzugsweise beträgt die Menge 1 bis 1,2 Äquivalente an Ameisensäure, bezogen auf ein Äquivalent NCO.

Die Umsetzung mit Ameisensäure erfolgt normalerweise ohne Verdünnungsmittel. Selbstverständlich kann diese Formylierungsreaktion auch in üblichen NCO-inerten Verdünnungsmitteln wie Kohlenwasserstoffen, Nitrilen und Ketonen durchgeführt werden, insbesondere bei Ausgangsisocyanaten höherer Viskosität, damit die Rührbarkeit des Reaktionsansatzes gewährleistet bleibt. Der Ablauf der exothermen Reaktion kann über die entwickelte Menge $CO_2$ verfolgt werden (pro NCO-Äquivalent 1 Mol $CO_2$). Die Reaktion wird im allgemeinen solange durchgeführt, bis die $CO_2$-Entwicklung beendet ist. Danach sind alle NCO-Gruppen in N-Formylgruppen der Formel

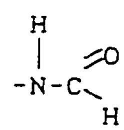

überführt, wie aus der NCO-Freiheit des IR-Spektrums ersichtlich ist. Die Formylierungsreaktion wird im allgemeinen innerhalb des Temperaturbereichs von 20 bis 150°C durchgeführt. Vorzugsweise liegt die Reaktionstemperatur im Bereich von 40 bis 100°C.

Die Aufarbeitung des nach Beendigung der Reaktion vorliegenden Reaktionsgemisches kann beispielsweise so erfolgen, daß niedrig siedende Anteile (Rest Ameisensäure, gegebenenfalls inerte Lösungsmittel) im Vakuum entfernt werden. Man erhält so als Destillationsrückstand die N-formylierten Amine bzw. Polyamine als Zwischenprodukte des erfindungsgemäßen Verfahrens in hoher Ausbeute und outer Reinheit ohne nennenswerte Nebenreaktionen.

Die zweite Stufe des erfindungsgemäßen Verfahrens besteht in der Überführung der N-Formylverbindungen in die erfindungsgemäßen Verfahrensprodukte. Diese zweite Stufe des erfindungsgemäßen Verfahrens erfolgt durch eine sogenannte Transformylierungsreaktion nach dem Schema

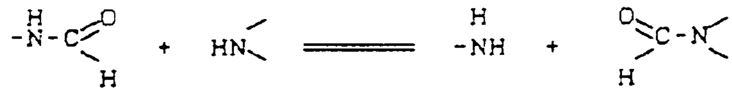

I II

Derartige Reaktionen sind bisher lediglich unter dem allgemeinen Begriff "Transamidierung" zur Herstellung von Formamiden beschrieben worden. So entsteht aus Hexamethylendiamin (HDA) und Formamid im Sinne obiger Gleichgewichtsreaktion monoformyliertes HDA in schlechter Qualität und nur bei Anwendung hoher Temperaturen und langer Reaktionszeiten (Synthesis (1988), S. 917-918). Entsprechende Transamidierungsreaktionen beschreibt EP-A 0 271 093 zur Herstellung von Formamiden aus Amin und Dimethylformamid unter Metalloxidkatalyse zur Verkürzung der Reaktionszeit.

Im Gegensatz zu den in diesen Vorveröffentlichungen beschriebenen Verfahren erfolgt beim erfindungsgemäßen Verfahren in glatter Reaktion ein Austausch der N-Formylgruppen durch primäre Aminogruppen, ohne daß lange Reaktionszeiten, extrem hohe Reaktionstemperaturen oder Katalysatoren zur Anwendung gelangen müßten.

Bei dieser Transformylierungsreaktion erfolgt eine Umsetzung der N-formylierten Zwischenprodukte mit primären oder sekundären Aminen, wobei eine Substitution der Formylgruppe

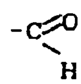

durch ein Wasserstoffatom erfolgt.

Für diese Umsetzung geeignete Amine sind beliebige primäre oder sekundäre Amine, wobei die Aminogruppen aliphatisch, cycloaliphatisch, aromatisch oder heterocyclisch gebunden sein können. Bevorzugt werden jedoch stark basische Amine eingesetzt, die bei 1013 mbar einen Siedepunkt von 80 bis 150°C, insbesondere 90 bis 130°C aufweisen. Zu den geeigneten und bevorzugt geeigneten Aminen gehören Alkylamine oder Dialkylamine mit 3 bis 18 Kohlenstoffatomen wie beispielsweise n-Propylamin, Methyl-ethylamin, n-Hexylamin, n-Dodecylamin oder n-Stearylamin, Alkylendiamine mit 2 bis 6, vorzugsweise 2 bis 4 Kohlenstoffatomen wie Ethylendiamin, 1,2- und 1,3-Diaminopropan, 1,4-, 1,2- oder 2,3-Diaminobutan oder Hexamethylendiamin oder heterocyclische Amine wie beispielsweise Pyrrolidin, Piperidin oder Morpholin. Besonders bevorzugt wird Ethylendiamin verwendet.

Bei der Durchführung der Transformylierungsreaktion werden vorzugsweise die Amine in einer solchen Menge eingesetzt, daß auf jede N-Formylgruppe 2 bis 25, vorzugsweise 6 bis 18 primäre und/oder sekundäre Aminogruppen des Amins entfallen.

Die Transformylierungsreaktion wird vorzugsweise lösungsmittelfrei und vorzugsweise innerhalb des Temperaturbereichs von 80 bis 150°C, insbesondere 90 bis 130°C unter Verwendung von Aminen eines entsprechenden Siedeverhaltens drucklos unter Rückflußbedingungen durchgeführt. Im allgemeinen ist die Umsetzung nach 1 bis 5 Stunden beendet Das Reaktionsgemisch wird anschließend destillativ, beispielsweise durch Dünnschichtdestillation aufgearbeitet, wobei die Verfahrensprodukte als Destillationsrückstand anfallen Auf diese Weise ist es ohne weiteres möglich, zu Verfahrensprodukten zu gelangen, in denen nur noch geringe Restmengen an N-Formylgruppierungen vorliegen. Die Konzentration an diesen N-Formylgruppen kann im übrigen auf einfache Weise durch Verwendung eines hohen Aminüberschusses bei der Transformylierungsreaktion innerhalb der genannten Bereiche minimiert werden Grundsätzlich ist es jedoch auch möglich, zur Minimierung des Restgehalts an N-Formylgruppen im Destillationsrückstand diesen in einer zweiten Reaktion nochmals mit einem Amin der beispielhaft genannten Art im Sinne einer Transformylierungsreaktion umzusetzen und das dabei anfallende Reaktionsgemisch erneut aufzuarbeiten.

Bei Verwendung von Isocyanuratgruppen aufweisenden Polyisocyanatgemischen, die im wesentlichen aus

a) 40 bis 90 Gew.-% N,N',N"-Tris-(6-isocyanatohexyl)isocyanurat und

b) 10 bis 60 Gew.-% an höheren Homologen dieses Triisocyanats

bestehen, und die, wie bereits oben ausgeführt, die besonders bevorzugten Ausgangsmaterialien für das erfindungsgemäße Verfahren darstellen, werden als erfindungsgemäße Verfahrensprodukte Isocyanuratgruppen aufweisende Polyamine erhalten, die bezüglich ihrer molaren Homologenverteilung den als Ausgangsmaterial eingesetzten Polyisocyanatgemischen entsprechen.

Diese erfindungsgemäßen Polyamingemische bestehen daher im wesentlichen aus

a) N,N',N''-Tris-(6-aminohexyl)-isocyanurat und

b) höheren, mehr als einen Isocyanuratring aufweisenden Homologen dieses Triamins,

wobei in erster Näherung davon ausgegangen werden kann, daß, bezogen auf das Gesamtgewicht der Komponenten a) und b), in dem Gemisch 40 bis 90 Gew.-% der Komponente a) und 10 bis 60 Gew.-% der Komponente b) vorliegen.

Die Homologenverteilung in den besonders bevorzugten erfindungsgemäßen Ausgangsmaterialien und in den erfindungsgemäßen Polyamingemischen kann gelchromatographisch ermittelt werden. Die erfindungsgemäßen Polyamingemische weisen einen Aminstickstoffgehalt (berechnet als N, Atomgewicht = 14) von 6 bis 9 Gew.-% auf.

Auch bei Verwendung von NCO-Prepolymeren, beispielsweise auf Basis von organischen Diisocyanaten und unterschüssigen Mengen an Polyetherpolyolen werden beim erfindungsgemäßen Verfahren Verfahrensprodukte einer den Prepolymeren analogen Struktur erhalten, ohne daß merkliche Anteile der Urethanbindungen zerstört sind.

Die erfindungsgemäßen Polyamingemische stellen wertvolle Härter für Epoxid- und insbesondere Isocyanatgruppen aufweisende Kunststoffvorläufer darf.

Epoxidgruppen aufweisende Kunststoffvorläufer, die mit den erfindungsgemäßen Polyaminen ausgehärtet werden können, sind beliebige organische Epoxidharze wie sie beispielsweise in Ullmann's Encyclopedia of Industrial Chemistry, Fifth Edition, Vol. A 9, 547 - 563, VCH Verlagsgesellschaft, Weinheim (1987) beschrieben sind und beispielsweise als Klebstoffe oder Beschichtungsmittel Verwendung finden.

Zu den Isocyanatgruppen aufweisenden Kunststoffvorläufern, die mit den erfindungsgemäßen Polyamingemischen ausgehärtet werden können, gehören insbesondere die aus der Polyurethanchemie an sich bekannten NCO-Prepolymeren, wie sie beispielsweise bereits oben unter 3. als Ausgangsmaterial für das erfindungsgemäße Verfahren angesprochen wurden oder auch solche NCO-Prepolymere, die durch Umsetzung von überschüssigen Mengen der beispielhaft genannten Diisocyanate mit Gemischen aus organischen Polyhydroxylverbindungen und einwertigen Alkoholen erhältlich sind. Derartige NCO-Prepolymere werden beispielsweise bei der Herstellung von wäßrigen Polyurethandispersionen verwendet, wie dies nachstehend in Beispiel 3 erläutert wird.

In den nachstehenden Beispielen beziehen sich alle Prozentangaben auf Gewichtsprozente.

<u>Beispiel 1</u>

a) <u>Formylierung eines Polyisocyanatgemischs</u>

In ein Gemisch aus 400 g eines handelsüblichen Trimerisats von Hexamethylendiisocyanat, bestehend im wesentlichen aus 51 % N,N',N''-Tris-(isocyanatohexyl)-isocyanurat und 49 % an höheren Homologen dieses Triisocyanats (NCO-Gehalt des Polyisocyanatgemischs: 21,5 %, Viskosität (24°C): 3.500 mPa.s) und 50 g Toluol wurden bei 50°C 100 g wasserfreie Ameisensäure so eingetropft, daß die Temperatur des Reaktionsansatzes bis 70°C ansteigt. Das Ende der Formylierungsraktion wurde durch die Beendigung der $CO_2$-Entwicklung und das Fehlen der NCO-Bande (IR) angezeigt. Man erhielt nach Abtrennung der Niedrigsieder im Vakuum auf diese Weise 406 g des dem Ausgangsgemisch entsprechenden N-formylierten Zwischenprodukts in Form einer wachsartigen, farblosen Substanz.

| <u>Analyse (%)</u>: | C | H | N |
|---|---|---|---|
| | 56,9 | 8,0 | 16,6 |

<u>Kernresonanzspektrum:</u>

$^1$H-NMR (200 MHz, DMSO) δ:

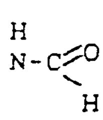

(breit 7,8 bis 8); $CH_2$ (breit 3,7); $CH_2$ (breit 3,1); $CH_2$ (breit 1,1 bis 1,7) (Verhältnisse: 1:1:1:4).

b) Herstellung eines Polyamingemischs

Das N-formylierte Zwischenprodukt aus Beispiel 1a) wurde in 1000 g Ethylendiamin 2 Stunden unter Rückflußbedingungen (Innentemperatur 120°C) gerührt. Nach Abtrennung der Siedeanteile (Ethylendiamin und Formylhomologes) durch Dünnschichtdestillation bei 140°C/0,1 mbar erhielt man 350 g eines klaren, hellgelben Polyamingemischs mit einem Aminstickstoffgehalt von 7,3 %.

Beispiel 2

Beispiel 1a) wird wiederholt. Das dabei erhaltene N-formylierte Zwischenprodukt wurde in 500 g Ethylendiamin 1 Stunde unter Rückflußbedingungen gerührt, entsprechend Beispiel 1 durch Dünnschichtdestillation von Siederanteilen befreit und das so erhaltene Sumpfprodukt nochmals in 240 g Ethylendiamin für 1 Stunde unter Rückflußbedingungen gerührt. Nach Aufarbeitung gemäß Beispiel 7 erhielt man 340 g eines gelben Polyamingemischs mit einem Aminstickstoffgehalt von 7,2 %.

Beispiel 3

a) Herstellung eines NCO-Prepolymeren

1680 g eines entwässerten Polyetherdiols der OH-Zahl 200, hergestellt durch Propoxylierung von Bisphenol A, wurden bei 60°C mit 3024 g 1,6-Diisocyanatohexan versetzt. Anschließend rührte man bei 100°C solange, bis ein NCO-Wert von 26,7 %. erreicht wurde (3 Stunden). Nach Abtrennung überschüssigen 1,6-Diisocyanatohexans durch Dünnschichtdestillation (150°C/0,1 mbar) erhielt man 2600 g NCO-Prepolymeres mit einem NCO-Gehalt von 8 %.

b) Formylierung und anschließende Transformylierung

In 890 g Prepolymeres gemäß Beispiel 3a) und 50 g Toluol wurden bei 50°C 83 g wasserfreie Ameisensäure so eingetropft, daß die Temperatur bis 70°C anstieg. Nach Beendigung der Formylierungsreaktion wurden Niedrigsieder im Vakuum abgetrennt, der Rückstand in 816 g Ethylendiamin gelöst und 1 Stunde unter Rückflußbedingungen (120°C) gerührt. Nach Aufarbeitung gemäß Beispiel 7 erhielt man ein farbloses, hochviskoses Polyamingemisch mit einem Aminstickstoffgehalt von 2,7 %.

Beispiel 4 (Verwendungsbeispiel)

a) Herstellung eine NCO-Prepolymeren

Ein Gemisch aus 289 g (0,17 Mol) eines Polyesterdiols des Molekulargewichts 1700 aus Adipinsäure und einem Gemisch aus 1,6-Dihydroxyhexan und Neopentylglykol im Gewichtsverhältnis 1,9:1 und 4,2 g eines einwertigen Polyetheralkohols des Molekulargewichts 2150, hergestellt durch Alkoxylierung von n-Butanol unter Verwendung eines Gemischs aus Ethylenoxid und Propylenoxid im Molverhältnis 80:20 als Alkoxylierungsmittel, wurde bei 100°C im Vakuum entwässert und bei 60°C mit 56,6 g (0,337 Mol) 1,6-Diisocyanatohexan versetzt. Anschließend wurde unter Rühren auf 105°C erwärmt und die Mischung während 2 h bei dieser Temperatur gehalten. Nach Abkühlen auf 60°C wurde in 150 g Aceton gelöst. Der NCO-Gehalt lag, bezogen auf Feststoff, bei 3,2 %.

b) Herstellung einer vernetzten Polyurethanharnstoffdispersion

400 g der Prepolymerlösung gemäß Beispiel 4a) wurden mit 710 ml Aceton verdünnt und unter Rühren bei 40°C mit einem Gemisch aus 1,3 g Isophorondiamin, 1,0 g Hydrazin und 10,0 g des Natriumsalzes der N-(2-Aminoethyl)-2-aminoethansulfonsäure, gelöst in 40 ml Wasser, versetzt. Nach 5 Min, wurden unter gutem Rühren 5,1 g des erfindungsgemäßen Polyamingemischs gemäß Beispiel 1b) zugegeben. Nach weiteren 10 Min, wurde mit 875 ml destilliertem Wasser bei 45°C dispergiert und das Aceton im Vakuum abdestilliert.

Man erhielt eine stabile wäßrige Polyurethanharnstoffdispersion mit folgenden Kenndaten:

| | |
|---|---|
| Festkörpergehalt: | 30,2 % |
| pH-Wert: | 7,0 |
| Harnstoffgruppen: | 3,8 % |
| Sulfonatgruppen: | 1,0 % |

Lösungsmittel: frei.

Ein aus der Dispersion gegossener Film wurde zunächst an der Luft (über Nacht) und dann während 30 Min. bei 130°C getrocknet. Man erhielt einen klebfreien, optisch klaren und glänzenden Film mit ausgezeichneter Haftung auf Glas und Aluminium.

Beispiel 5

Beispiel 4b) wurde wiederholt, jedoch nunmehr unter Verwendung von 0,6 g Diethylentriamin, 0,9 g Hydrazinhydrat und 20,9 g des erfindungsgemäßen Polyamingemisches gemäß Beispiel 3b).

Man erhielt eine stabile, vernetzte wäßrige Polyurethanharnstoffdispersion mit einem Festkörpergehalt von 32,1 % bei einem pH-Wert von 7,0. Die Dispersion ist in Dimethylformamid unlöslich, ebenso ein daraus gebildeter Beschichtungsfilm.

## Patentansprüche

1. Verfahren zur Herstellung von primären Aminen mit aliphatisch oder cycloaliphatisch gebundenen Aminogruppen durch Formylierung der Isocyanatgruppen von orgnanischen Isocyanaten mit aliphatisch oder cycloaliphatisch gebundenen Isocyanatgruppen mit Ameisensäure und anschließende Überführung der N-Formylgruppen in Aminogruppen, dadurch gekennzeichnet, daß man die N-Formylgruppen durch eine Transformylierungsreaktion in Aminogruppen überführt.

2. Verfahren gemäß Anspruch 1, dadurch gekennzeichnet, daß man als Ausgansmaterialien Gemische von Isocyanuratgruppen aufweisenden Polyisocyanaten verwendet, die im wesentlichen aus
   a) 40 bis 90 Gew.-%, bezogen auf das Gesamtgewicht der Komponenten a) und b), N,N',N"-Tris-(6-isocyanatohexyl)-isocyanurat und
   b) 10 bis 60 Gew.-%, bezogen auf das Gesamtgewicht der Komponenten a) und b), an höheren, mehr als einen Isocyanuratring aufweisenden Homologen dieses Triisocyanats bestehen.

3. Polyamingemische mit aliphatisch gebundenen Aminogruppen, dadurch gekennzeichnet, daß die sie im wesentlichen aus
   a) 40 bis 90 Gew.-%, bezogen auf das Gesamtgewicht der Komponenten a) und b), N,N',N"-Tris-(6-aminohexyl)-isocyanurat und
   b) 10 bis 60 Gew.-%, bezogen auf das Gesamtgewicht der Komponenten a) und b), an höheren, mehr als einen Isocyanurating aufweisenden Homologen dieses Triamins bestehen.

## Claims

1. A process for the production of primary amines containing aliphatically or cycloaliphatically bound amino groups by formylation of the isocyanate groups of organic isocyanates containing aliphatically or cycloaliphatically bound isocyanate groups with formic acid and subsequent conversion of the N-formyl groups into amino groups, characterized in that the N-formyl groups are converted into amino groups by a transformylation reaction.

2. A process as claimed in claim 1, characterized in that mixtures of isocyanurate polyisocyanates consisting essentially of
   a) 40 to 90% by weight, based on the total weight of components a) and b), N,N',N"-tris-(6-isocyanatohexyl)-isocyanurate and
   b) 10 to 60% by weight, based on the total weight of components a) and b), of higher homologs of this tri isocyanate containing more than one isocyanurate ring

   are used as the starting materials.

3. Polyamine mixtures containing aliphatically bound amino groups, characterized in that they consist essentially of
   a) 40 to 90% by weight, based on the total weight of components a) and b), N,N',N"-tris-(6-isocyanatohexyl)-isocyanurate and
   b) 10 to 60% by weight, based on the total weight of components a) and b), of higher homologs of this

triisocyanate containing more than one isocyanurate ring.

## Revendications

1. Procédé de production d'amines primaires portant des groupes amino en liaison aliphatique ou cycloaliphatique par formylation des groupes isocyanato d'isocyanates organiques porteurs de groupes isocyanato en liaison aliphatique ou cycloaliphatique avec l'acide formique, suivie de la transformation des groupes N-formyle en groupes amino, caractérisé en ce qu'on transforme les groupes N-formyle en groupes amino par une réaction de transformylation.

2. Procédé suivant la revendication 1, caractérisé en ce qu'on utilise comme matières de départ des mélanges de polyisocyanates porteurs de groupes isocyanurate, qui sont constitués
   a) de 40 à 90 % en poids, par rapport au poids total des composants a) et b), de N,N',N"-tris-(6-isocyanatohexyl)isocyanurate et
   b) de 10 à 60 % en poids, par rapport au poids total des composants a) et b), d'homologues supérieurs de ce triisocyanate, présentant plus d'un noyau isocyanurate.

3. Mélanges de polyamines portant des groupes amino en liaison aliphatique, caractérisés en ce qu'ils sont principalement constitués
   a) de 40 à 90 % en poids, par rapport au poids total des composants a) et b), de N,N',N"-tris-(6-aminohexyl)isocyanurate et
   b) de 10 à 60 % en poids, par rapport au poids total des composants a) et b), d'homologues supérieurs de cette triamine, présentant plus d'un noyau isocyanurate.